(19) Europäisches Patentamt / European Patent Office / Office européen des brevets

(11) **EP 3 100 039 B1**

(12) **EUROPÄISCHE PATENTSCHRIFT**

(45) Veröffentlichungstag und Bekanntmachung des Hinweises auf die Patenterteilung:
**02.12.2020 Patentblatt 2020/49**

(21) Anmeldenummer: **15706361.1**

(22) Anmeldetag: **29.01.2015**

(51) Int Cl.:
**G01N 27/414** (2006.01)   **G01N 33/487** (2006.01)

(86) Internationale Anmeldenummer:
**PCT/DE2015/100040**

(87) Internationale Veröffentlichungsnummer:
**WO 2015/113560 (06.08.2015 Gazette 2015/31)**

(54) **VORRICHTUNG UND VERFAHREN ZUR MESSUNG BIOLOGISCHER UND/ODER ELEKTRONISCHER EIGENSCHAFTEN EINER PROBE SOWIE VERWENDUNGEN DERSELBEN**

DEVICE AND METHOD FOR MEASURING BIOLOGICAL AND/OR ELECTRONIC PROPERTIES OF A SAMPLE, AND USES THEREOF

DISPOSITIF DE MESURE DE PROPRIÉTÉS BIOLOGIQUES ET/OU ÉLECTRONIQUES D'UN ÉCHANTILLON AINSI QUE DES UTILISATIONS DE CEUX-CI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.01.2014 DE 102014001155**

(43) Veröffentlichungstag der Anmeldung:
**07.12.2016 Patentblatt 2016/49**

(73) Patentinhaber: **Hochschule Kaiserslautern**
**67657 Kaiserslautern (DE)**

(72) Erfinder:
• **INGEBRANDT, Sven**
  **55232 Alzey (DE)**
• **SUSLOPAROVA, Anna**
  **76135 Karlsruhe (DE)**
• **VU, Xuan, Thang**
  **52070 Aachen (DE)**
• **KOPPENHÖFER, Dieter**
  **66482 Zweibrücken (DE)**
• **KA-YAN LAW, Jessica**
  **66482 Zweibrücken (DE)**

(74) Vertreter: **Manasse, Uwe**
**Boehmert & Boehmert**
**Anwaltspartnerschaft mbB**
**Pettenkoferstrasse 22**
**80336 München (DE)**

(56) Entgegenhaltungen:
WO-A1-2008/061489

• SPANU A ET AL: "Organic FET device as a novel sensor for cell bioelectrical and metabolic activity recordings", 2013 6TH INTERNATIONAL IEEE/EMBS CONFERENCE ON NEURAL ENGINEERING (NER), IEEE, 6. November 2013 (2013-11-06), Seiten 937-940, XP032538402, ISSN: 1948-3546, DOI: 10.1109/NER.2013.6696089 [gefunden am 2013-12-26]
• CLÉMENT SUSPÈNE ET AL: "Copolythiophene-based water-gated organic field-effect transistors for biosensing", JOURNAL OF MATERIALS CHEMISTRY B, Bd. 1, Nr. 15, 1. Januar 2013 (2013-01-01), Seite 2090, XP055182070, ISSN: 2050-750X, DOI: 10.1039/c3tb00525a
• NARAYAN K S ET AL: "Water-Gated Phospholipid-Monolayer Organic Field Effect Transistor Through Modified Mueller Montal Method", IEEE ELECTRON DEVICE LETTERS, IEEE SERVICE CENTER, NEW YORK, NY, US, Bd. 34, Nr. 2, 1. Februar 2013 (2013-02-01), Seiten 310-312, XP011488951, ISSN: 0741-3106, DOI: 10.1109/LED.2012.2233192
• LOIG KERGOAT ET AL: "A Water-Gate Organic Field-Effect Transistor", ADVANCED MATERIALS, Bd. 22, Nr. 23, 20. Mai 2010 (2010-05-20), Seiten 2565-2569, XP055182045, ISSN: 0935-9648, DOI: 10.1002/adma.200904163

**Beschreibung**

Die vorliegende Erfindung betrifft eine Vorrichtung zur Messung biologischer und/oder elektronischer Eigenschaften einer Probe, insbesondere zur Messung von Zellparametern insbesondere einzelner menschlicher, pflanzlicher oder tierischer Zellen, ein Array aus mindestens zwei derartigen Vorrichtungen, einen Sensor-Chip, ein Verfahren zur Messung biologischer und/oder elektronischer Eigenschaften einer Probe, insbesondere zur Messung von Zellparametern insbesondere einzelner menschlicher, pflanzlicher oder tierischer Zellen, Verwendungen der Vorrichtung und des Verfahrens sowie ein Zell-Chip-Hybridsystem (Zell-Chip-Hybridtestsystem) umfassend einen Feldeffekttransistor mit einer Kontaktierungseinrichtung zur Kontaktierung der Probe mit dem Gate des Feldeffekttransistors und eine Stimulationseinrichtung zur Beaufschlagung der Probe mit einer elektrischen Wechselspannung zur Impedanzspektroskopie.

Zur Untersuchung von Zellaktivitäten in Gewebekulturen stehen im Wesentlichen drei kommerzielle Systeme zur Verfügung. Dabei handelt es sich um das ECIS-System der Firma Applied Biophysics, das Cellkey-System der Firma Molecular Devices und das xCELLigence™-System der Firma ACEA Biosciences. Das Messprinzip der bekannten Systeme beruht auf der Impedanzmessung an metallischen Mikro- und Makroelektroden. Ein wesentlicher Nachteil der auf dem Markt befindlichen Geräte stellt die geringe laterale Auflösung, das heißt im Wesentlichen die relativ großen Sensorelektroden in relativ großen Abständen dar. Mit anderen Worten ist mit den bekannten kommerziellen Geräten keine echte Einzelzellanalyse möglich, was die nutzbaren Zelltypen und auch die möglichen biomedizinischen Analysen stark einschränkt.

Aus der WO 2008/061489 A1 sind eine Vorrichtung und ein Verfahren zur Messung biologischer und elektronischer Eigenschaften einer Probe bekannt. Die bekannte Vorrichtung (Sensor) umfasst einen Feldeffekttransistor mit Kontaktierungsmitteln zur Kontaktierung der Probe mit dem Gate des Feldeffekttransistors sowie Stimulationsmittel zur Beaufschlagung der Probe mit einer Wechselspannung. Das bekannte Verfahren weist die Schritte auf: die Probe wird mit einer Wechselspannung beaufschlagt; die Probe tauscht Ladungen mit dem Gate eines Feldeffekttransistors aus und eine vom Potenzial am Gate des Feldeffekttransistors abhängige primäre physikalische Messgröße wird gemessen. Zumindest in einer besonderen Ausführungsform dient das bekannte Verfahren zur Impedanzspektroskopie.

1 SPANU A ET AL: "Organic FET device as a novel sensor for cell bioelectrical and metabolic activity recordings",2013 6TH INTERNATIONAL IEEE/EMBS CONFERENCE ON NEURAL ENGINEERING (NER), IEEE, 6. November 2013 (2013-11-06), Seiten 937-940 offenbart eine Vorrichtung zur Messung von Zellparametem einzelner menschlicher, pflanzlicher oder tierischer Zellen, mit einem organischen Feldeffekttransistor (OCMFET) der eine Kontaktierungseinrichtung zur Kontaktierung der Probe mit dem Gate des Feldeffekttransistors und eine Stimulationseinrichtung zur Beaufschlagung der Probe mit einer elektrischen Wechselspannung aufweist, zur Messung eines Aktionspotentials mittels einer Schaltung zur Strom-Spannungswandlung.

Mittels der aus der WO 200/061489 A1 bekannten Vorrichtung lässt sich eine echte Einzelzellanalyse vornehmen. Dies wiederum lässt eine wesentlich breitere Anwendung und die Nutzung von anderen Zellkulturen, die in der Regel keine geschlossenen Zellschichten bilden, wie zum Beispiel Nervenzellen, Einzelzellen des Immunsystems und ähnliches zu. Die bekannten Sensoren auf Basis von Silizium-Feldeffekt-Transistoren sind jedoch nur aufwendig und damit teuer herzustellen und haben den wesentlichen Nachteil, dass sie nicht optisch transparent sind.

Der vorliegenden Erfindung liegt somit die Aufgabe zu Grunde, die bekannte Vorrichtung so weiterzubilden, dass sie kostengünstiger herstellbar ist.

Erfindungsgemäß wird diese Aufgabe durch eine Vorrichtung entsprechend dem unabhängigen Anspruch 1 bzw. durch ein Verfahren entsprechend dem unabhängigen Anspruch 13 gelöst, wobei der Feldeffekttransistor ein organischer Feldeffekttransistor (OFET) ist, vorzugsweise wobei der Feldeffekttransistor einen für das Messverfahren optimierten Frequenzbereich zur Impendanzspektroskopie aufweist.

Insbesondere kann dabei vorgesehen sein, dass der Feldeffekttransistor ein Gate-Breite W/Gate-Länge L-Verhältnis (W/L-Verhältnis) im Bereich von 1 bis 10000, vorzugsweise im Bereich von 10 bis 1000 und besonders bevorzugt im Bereich von 100 bis 1000 aufweist. Je größer der Wert des W/L-Verhältnisses ist, desto höher ist üblicherweise die Empfindlichkeit und damit das Signal-zu-Rausch Verhältnis (S/N-Verhältnis). Darüber hinaus steigt mit dem W/L-Verhältnis die Bandbreite der Messung, was insbesondere für die aus der WO 200/061489 A1 bekannte Methode vorteilhaft ist.

Vorteilhafterweise weist der Feldeffekttransistor eine Fingerstruktur zur Steigerung des oben genannten W/L Verhältnisses auf. Beispielweise können die Source- und Drain-Elektroden kammförmig gestaltet und verzahnt angeordnet sein. Dadurch kann der zur Verfügung stehende Platz maximal oder optimal ausgenutzt werden unter (zusätzlicher) Minimierung der Gate-Länge L. Ziel ist aber ebenfalls, den zur Verfügung stehenden Platz maximal beziehungsweise optimal auszunutzen unter Minimierung der Gate-Länge L. Demzufolge ist es von Vorteil, wenn der Feldeffekttransistor eine Struktur zur Vergrößerung des W/L-Verhältnisses aufweist.

Vorteilhafterweise ist die Messfläche des Feldeffekttransistors kleiner als $400 \mu m^2$ und/oder ist der Durchmesser der Messfläche kleiner als circa $20 \mu m$ und/oder ist die gesamte Messfläche oder nur ein Teil derselben optisch transparent. Wenn die Messfläche beispielsweise quadratisch ist, so liegt die Seitenlänge in etwa im Bereich des oberen Durchmessers einer einzelnen Zelle. Metallelektroden der oben genannten, kommerziellen Systeme müssen aus messtechnischen Gründen viel größer als einzelne Zellen sein. Damit werden immer nur kollektive, gemittelte Antworten vieler Zellen vermessen. Das geht in der Zellkulturtechnik mit sogenannten Zelllinien gut, bei denen alle Zellen Klone einer Mutterzelle sind und die eine kollektive Antwort geben. Eine Biopsie eines Patienten, eine Kultur von Stammzellen oder eine primäre Zellkultur von Nervenzellen ist jedoch heterogen und besteht aus verschiedenen Zelltypen, die unterschiedliche Morphologien und Eigenschafen zeigen. Da hilft die Miniaturisierung auf Einzelzellen bei gleichzeitiger Nutzung von sehr vielen, gleichartigen Sensoren auf einem Chip. Somit hat man mit einem solchen Chip auch die Möglichkeit, alle Daten zu mitteln und die gleichen Aussagen zu erhalten wie bei den Standardsystemen. Man erhält jedoch durch die hier beschriebene Messmethode zusätzlich die Information über die Zell-zu-Zell-Variabilität, die von sehr großem Wert für die oben genannten und im Folgenden weiter ausgeführten Anwendungen an Biopsien oder Stammzellkulturen ist

Wenn die gesamte Messfläche oder nur ein Teil der derselben optisch transparent ist, so kann die Vorrichtung auch in Kombination mit Vorrichtungen zur Durchführung von (anderen) optischen Untersuchungsverfahren, die standardmäßig in der Zellkulturtechnik verwendet werden. Zudem lässt die optische Transparenz und die Möglichkeit zur extrem kostengünstigen Herstellung und damit eine Einwegverwendung der Sensoren als sogenannte Disposables eine breite Akzeptanz im biomedizinischen Markt und in der Zellkulturtechnologie erwarten.

Zum Erreichen der notwendigen Bandbreite der Sensoren und zur Realisierung der Impedanzmessmethode sollte vorteilhafterweise die Gate-Länge L des Transistors kleiner als circa 1 μm sein. Noch allgemeiner ist es zum Erreichen des benötigten S/N-Verhältnisses von Vorteil, wenn die Gate-Länge L kleiner als die Auflösungsgrenze der optischen Lithografie (ca. 1 μm) ist. Dies könnte z. B. mittels eines geeigneten Nanostrukturierungsverfahrens, wie z. B. Elektronenstrahllithografie, deep-UV-Lithografie oder Nanoimprintlithografie, erreicht werden.

Günstigerweise ist der Feldeffekttransistors mittels eines Nanostrukturierungsverfahrens, zum Beispiel Elektronenstrahllithografie, UV-Lithografie oder Nanoimprintlithografie, strukturiert.

Vorteilhafterweise bestehen die Source- und Drain-Zuleitungen aus einem elektrisch leitfähigen, vorzugsweise optisch transparenten Material oder einer entsprechenden Materialmischung und sind mit einer Passivierungsschicht, wie z. B. $SiO_2$, $Si_3N_4$ oder Parylene-C, versehen, insbesondere elektrisch isoliert. Dadurch lassen sich die parasitären Kapazitäten der Zuleitungen reduzieren beziehungsweise geeignet einstellen.

Günstigerweise ist das organische Halbleitermaterial des Feldeffekttransistors ein optisch transparentes Polymer, (wie z. B. PEDOT:PSS), ein Oligomer, ein 2D-Material (wie z. B. Graphen oder reduziertes Graphenoxid), ein Chalcogenid (wie z. B. $MoS_2$), oder eine Kombination oder Mischung von mehreren derselben. Ganz allgemein ist es von Vorteil, wenn das Halbleitermaterial kostengünstig und/oder druckbar ist. Erfindungsgemäß weist das organische Material des Feldeffekttransistors eine Ladungsträgerbeweglichkeit $\mu$ von 0.1 cm²/Vs bis 3,6 cm²/Vs auf. Beispielsweise weist PEDOT:PSS (Poly-3, 4-Ethylendioxythiophen) eine für halbleitende Polymere relativ hohe typische Ladungsträgerbeweglichkeit von 0,1cm²/Vs auf. Es wurde bereits beschrieben, dass durch die Nutzung von Ethylenglycol in Kombination mit PEDOT:PSS sich die Ladungsträgerbeweglichkeit auf 3,6 cm²/Vs erhöhen lässt (M. Yamashita, C. Otani, M. Shimizu, and H. Okuzaki, Effect of solvent on carrier transport in poly(3,4-ethylenedioxythiophene)/poly(4-styrenesulfonate) studied by terahertz and infraredultraviolet spectroscopy, Appl. Phys. Lett. 99, 143307 (2011)).

Im Zusammenhang mit beispielsweise PEDOT:PSS ist auch zu erwähnen, dass Zellen in ihrer natürlichen Umgebung normalerweise nicht an harten, technischen Materialen adhärieren (müssen), sondern eher weichere Materialien bevorzugen. Mit beispielsweise PEDOT:PSS kann bei der Herstellung von Sensoren auch auf weichen Oberflächen gearbeitet werden. Grundsätzlich wird die polymere Elektronik auf dem Fachgebiet allgemein als zu "langsam" angesehen, um als Transistoren für die Impedanzspektroskopie eingesetzt zu werden. Vor den Erfindern wurde jedoch in Experimenten erkannt, dass die passiven "Dämpfungseffekte" der Zellen auch schon bei niedrigeren Frequenzen sichtbar werden, sodass die Impedanzmethode, wie von Metallelektroden und Si-FETs bekannt, auch auf Polymer-FETs angewendet werden kann. Ein großer Vorteil ist dabei die Verschiebung der Effekte in den niedrigen Frequenzbereich. Eine Erklärung der Messeffekte ist weiter unten beschrieben.

Zweckmäßigerweise ist das organische Halbleitermaterial zur elektrischen Stabilisierung des Sensormaterials mit einer organischen Schicht, z. B. Ethylenglycol im Falle von PEDOT:PSS als Halbleitermaterial, behandelt. Dies dient sowohl zur Verbesserung der elektrischen Eigenschaften, aber auch zum Schutz vor Degradation durch das flüssige Messmedium, wie z. B. Zellmedium, Elektrolyt, Wasser. Wie oben beschrieben, wirkt sich eine solche Behandlung zusätzlich positiv auf eine höhere Ladungsträgerbeweglichkeit aus, was beim beschriebenen Messverfahren die Bandbreite und damit die Sensitivität erhöht.

Ferner weist die Vorrichtung vorzugsweise ein Substrat für den Feldeffekttransistor aus einem vorzugsweise optisch transparenten Material, wie z. B. Glas oder vorzugsweise optisch transparentes, vorzugsweise weiches Polymermaterial, wie z. B. Kapton, Neopulim® oder ähnlichem, oder aus einer Kombination derselben auf.

Dabei sind wie oben beschrieben weichere Materialien vorteilhaft.

Gemäß einer besonderen Ausführungsform der vorliegenden Erfindung ist die Vorrichtung als ein Sensor-Chip oder als ein Bestandteil desselben ausgebildet. Es können auch nur Teile der Vorrichtung als ein Sensor-Chip oder als Bestandteil desselben ausgebildet sein.

Die erfindungsgemäße Vorrichtung ist gekennzeichnet durch ein frequenzselektives Messinstrument zur frequenzabhängigen Messung von Amplitude und Phase eines durch die Source-Drain-Strecke des Feldeffekttransistors fließenden Stroms (Impendanzspektroskopie).

Wiederum eine weitere besondere Ausführungsform der erfindungsgemäßen Vorrichtung ist gekennzeichnet durch ein Anregungsmittel zur Beaufschlagung der Probe mit einer weiteren elektrischen Spannung oder einem elektrischen Strom.

Weiterhin liefert die vorliegende Erfindung ein Array aus mindestens zwei Vorrichtungen nach einem der Ansprüche 1 bis 13.

Darüber hinaus liefert die vorliegende Erfindung einen Sensor-Chip mit mindestens einer Vorrichtung nach einem der Ansprüche 1 bis 13.

Weiterhin wir diese Aufgabe gelöst durch ein Verfahren zur Messung biologischer und/oder elektronischer Eigenschaften einer Probe, insbesondere zur Messung von Zellparametern insbesondere einzelner menschlicher, pflanzlicher oder tierischer Zellen, umfassend die Schritte:

- Anordnen einer Probe in Kontakt mit dem Gate eines organischen Feldeffekttransistors insbesondere einer Vorrichtung nach einem der Ansprüche der 1 bis 13,

- Beaufschlagen der Probe mit einer Wechselspannung oder einem Wechselstrom und

- Messen einer vom Potenzial am Gate des Feldeffekttransistors abhängigen insbesondere primären physikalischen Messgröße. Vorzugsweise steht die Probe in direktem elektrischem Kontakt mit dem Gate des organischen Feldeffekttransistors.

Gemäß einer besonderen Ausführungsform der vorliegenden Erfindung weist die Wechselspannung eine Frequenz zwischen 0,1Hz und 1GHz, insbesondere zwischen 1Hz und 10MHz, auf und/oder weist die Wechselspannung eine Amplitude zwischen 0,001mV und 10V, insbesondere zwischen 1mV und 100mV, auf.

Weiterhin kann vorgesehen sein, dass ein durch die Source-Drain-Strecke des Feldeffekttransistors fließender Strom als physikalische Messgröße gemessen wird.

Vorteilhafterweise werden die Amplitude und Phase der physikalischen Messgröße gemessen.

Günstigerweise wird das Verfahren ort- und/oder zeitaufgelöst durchgeführt.

Des Weiteren ist die vorliegende Erfindung auf die Verwendung der Vorrichtung nach einem der Ansprüche 1 bis 10, des Arrays nach Anspruch 11 oder des Sensor-Chips nach Anspruch 12 zur impedimetrischen Analyse, insbesondere im Hochdurchsatz- und/oder Einzelzellverfahren, adhärenter Zellen in vitro gerichtet.

Insbesondere kann dabei vorgesehen sein, dass die Zelladhäsion oder Zelladhäsionsfähigkeit sowie die strukturelle Integrität der Zelle analysiert wird. Die Zelladhäsion beziehungsweise Zelladhäsionsfähigkeit kann als ein Indikator für die Zellviabilität angesehen beziehungsweise verwendet werden. Die strukturelle Integrität ist ein Maß dafür, ob eine intakte Zelle oder eine absterbende bzw. bereits abgestorbene Zelle vorliegt.

Darüber hinaus ist die vorliegende Erfindung nach einem der Ansprüche 1 bis 10, des Arrays nach Anspruch 11 oder des Sensor-Chips nach Anspruch 12 zur elektronischen Analyse, insbesondere impedimetrischen Analyse, insbesondere im Hochdurchsatzverfahren von Stammzellendifferenzierung gerichtet.

Weiterhin ist die vorliegende Erfindung auf die Verwendung der Vorrichtung nach einem der Ansprüche 1 bis 10, des Arrays nach Anspruch 11 oder des Sensor-Chips nach Anspruch 12 zur elektronischen Biopsie insbesondere im Hochdurchsatzverfahren von menschlichen, pflanzlichen oder tierischen Geweben, insbesondere Tumorgewebe gerichtet. Beispielsweise kann sie zur Tumordiagnose verwendet werden.

Ferner ist die vorliegende Erfindung auf die Verwendung der Vorrichtung nach einem der Ansprüche 1 bis 10, des Arrays nach Anspruch 11 und des Sensor-Chips nach Anspruch 12 zur Analyse der Wirkungsweise von pharmazeutischen Substanzen und Medikamenten, insbesondere Krebsmedikamenten, wie zum Beispiel Zytostatika und Chemotherapeutika oder Substanzen, die die Differenzierung von Stammzellen beeinflussen, gerichtet.

Darüber hinaus geht es bei der vorliegenden Erfindung um die Verwendung eines Verfahrens nach einem der Ansprüche 13 bis 15 zur elektronischen Analyse, insbesondere impedimetrischen Analyse, insbesondere im Hochdurchsatzverfahren von Stammzellendifferenzierung, die Verwendung eines Verfahrens nach einem der Ansprüche 13 is 15 30 zur elektronischen Biopsie insbesondere im Hochdurchsatzverfahren von menschlichen, pflanzlichen oder tierischen Geweben, insbesondere Tumorgewebe, und die Verwendung eines Verfahrens nach einem der Ansprüche 16 bis 20 zur Analyse der Zytotoxizität von Substanzen und Medikamenten, insbesondere Krebsmedikamenten, wie zum Beispiel Zytostatika und Chemotherapeutika.

Die vorliegende Erfindung ist ferner auf die Verwendung eines Verfahrens nach einem der Ansprüche 13 bis 15 in Kombination mit einem Durchlichtmessverfahren, wie zum Beispiel Durchlichtmikroskopieverfahren, gerichtet. In analoger Weise können die Verwendungen gemäß den Ansprüchen 16 bis 22 auch in Kombination mit einem Durchlichtmessverfahren erfolgen.

Schließlich liefert die vorliegende Erfindung auch ein Zell-Chip-Hybridsystem zur Überwachung der Aktivität von menschlichen und tierischen Zellen und zur Analyse der Zytotoxizität von Substanzen und Medikamenten, insbesondere Krebsmedikamenten, wie zum Beispiel Zytostatika und zur elektronischen Biopsie insbesondere im Hochdurchsatzverfahren von menschlichen und tierischen Geweben, insbesondere Tumorgewebe, und zur elektronischen Analyse, insbesondere impedimetrischen Analyse, insbesondere im Hochdurchsatzverfahren von Stammzellendifferenzierung, entsprechend dem Anspruch 24.

Der vorliegenden Erfindung liegt die überraschende Erkenntnis zu Grunde, dass durch die Verwendung von organischen Feldeffekttransistoren die Herstellungskosten der einzelnen Vorrichtungen beziehungsweise Sensoren soweit gesenkt werden können, dass ein Einsatz z. B. zum Standard Screening von Zellkulturen im medizinischen Massenmarkt in Form von Einweg-Sensoren möglich erscheint. Beispielsweise können die Herstellungskosten durch Verwendung von modernen Druckverfahren der Polymerelektronik deutlich gesenkt werden. Es ist auch eine massentaugliche Herstellung zum Beispiel durch Rollendruck denkbar. Aufgrund der deutlich niedrigeren Herstellungskosten können die Vorrichtungen beziehungsweise Sensoren auch als Wegwerfvorrichtungen beziehungsweise -sensoren vorgesehen und verwendet werden.

Wenn zumindest gemäß besonderen Ausführungsformen die Messfläche oder ein Teil derselben beziehungsweise die gesamte Vorrichtung optisch transparent ist, so wird dadurch eine optische Kontrolle der Zellkulturen und eine eventuelle Verbindung mit standardisierten optischen Untersuchungsmethoden der Zellbiologie möglich. Transparente Vorrichtungen beziehungsweise Sensoren liefern hier einen großen Vorteil, da die meisten Zellkulturlabore und biomedizinischen Analyselabore mit Durchlichtmikroskopie arbeiten. Wenn die Vorrichtung beispielsweise zur Impedanzspektroskopie benutzt wird, so lässt diese Technologie im Vergleich zu den bekannten Systemen eine echte Einzelzellanalyse zu, die eine wesentlich breitere Anwendung und die Nutzung von anderen Zellkulturen, die in der Regel keine geschlossenen Zellschichten bilden (zum Beispiel Nervenzellen, Einzelzellen des Immunsystems etc.) zulässt.

Die vorliegende Erfindung lässt zumindest in einer besonderen Ausführungsform die Nutzung von neuartigen Materialen der Polymerelektronik zu und kann die FET-Impedanzmethode konkurrenzfähig zu denen auf den Markt vorhandenen Geräten machen. Mit den impedimetrischen Verfahren gemäß der vorliegenden Erfindung lassen sich durch eine Impedanzanalyse im Wesentlichen die passiven Eigenschaften von Zellen abfragen, so dass dies mit allen Zelltypen funktioniert. Es können Rückschlüsse auf Adhäsionsstärke der Zelle und Integrität der Zellmembran gezogen werden, die z.B. Hinweise auf Interaktion der Zellen mit auf den Sensoren aufgebrachten Beschichtungen, Vorgänge bei der Differenzierung von Stammzellen in andere Zelltypen oder Reaktionen von Zellen auf chemische, pharmakologische oder physikalische Reize zulassen. Bei den impedimetrischen Verfahren werden Wechselstromsignale gemessen, d.h. dass eine Wechselspannung angelegt und eine Wechselspannung gemessen wird. Dies erfolgt als eine Frequenzanalyse. Mit anderen Worten wird das vorzugsweise komplette Spektrum vieler Frequenzen zur Analyse benutzt. Es wird eine Frequenzanalyse der Übertragungsstrecke (Transferfunktion) durchgeführt.

Zumindest in einer besonderen Ausführungsform der Erfindung kann in einer Zellkulturschale die Adhäsion einzelner Zellen gemessen werden. Insbesondere kann auch die Reaktion der Zellen auf zum Beispiel Zugabe von Medikamenten oder Cytotoxika getestet werden. Im Wesentlichen können durch eine hohe Integrationsdichte vieler FET-Vorrichtungen beziehungsweise -Sensoren in einer Zellkulturschale - mehr Daten - auch von verschiedenen Gewebearten - vermessen werden, als es momentan mit den auf dem Markt befindlichen Systemen möglich ist.

Zumindest in besonderen Ausführungsformen der Erfindung können die Vorrichtung und/oder das Verfahren beispielsweise zur histologischen Analyse von Tumorgewebe zur personalisierten Diagnostik und Therapieplanung bei Krebspatienten verwendet werden. In Deutschland wird bei fast einer halben Million Menschen jährlich Krebs diagnostiziert, wobei laut dem deutschen Krebsforschungsinstitut Darm-, Prostata-, Brust- und Lungenkrebs die häufigsten Diagnosen darstellen. Zur Behandlung werden komplexe Abfolgen bestehend aus Chemo- und Radiotherapie in Kombination mit Tumorresektion angewandt. Im Falle der Chemotherapie besteht allerdings bisher nicht die Möglichkeit, die Wirksamkeit der eingesetzten Medikamente im Vorfeld zu überprüfen, was zu einer erheblichen Belastung durch mögliche Nebenwirkungen ohne Therapieerfolg führen kann. Die vorliegende Erfindung liefert zumindest in besonderen Ausführungsformen eine Plattform, die zur Vorabtestung geplanter Behandlungskonzepte genutzt werden kann, um Wirksamkeit und Verträglichkeit individueller Chemotherapie zu optimieren. Dadurch wird sowohl der Leidensdruck der betroffenen Patienten gelindert als auch die Chance auf eine erfolgreiche Behandlung erhöht.

Des Weiteren kann beziehungsweise können die Vorrichtung und/oder das Verfahren zumindest in einer besonderen Ausführungsform beispielsweise für pharmakologische Studien zur Entwicklung selektiver Chemotherapeutika im Hochdurchsatz-Verfahren verwendet werden. Bisher zur Therapie bösartiger Tumore eingesetzte Wirkstoffe zeichnen sich durch eine verhältnismäßig geringe Selektivität aus und wirken unspezifisch auf Gewebe mit hohen Proliferationsraten.

Des Weiteren kann beziehungsweise können die Vorrichtung und/oder das Verfahren zumindest in besonderen Ausführungsformen beispielsweise zur Zelldifferenzierungs- und Stammzellenanalyse verwendet werden. Die Fähigkeit von Stammzellen zur Differenzierung in diverse Zelltypen stellt die Grundlage für das menschliche Immunsystem dar und ist ein hochkomplexes Forschungsgebiet. So ist zum Beispiel bei der Behandlung von Leukämie oder myelodysplastischen Syndromen die Transplantation von Stammzellen des hämatopoäthischen Systems langfristig betrachtet die einzig vielversprechende Behandlungsmethode. Die vorliegende Erfindung kann zumindest in besonderen Ausführungsformen dazu eingesetzt werden, die Differenzierung von Stammzellen in ihre Tochterzellen zu überwachen und zu analysieren. Die dabei stattfindenden morphologischen Veränderungen können mit Hilfe der beschriebenen Erfindung analysiert und so der Übergang der einzelnen Stammzelle in ihre Tochterzelle nachverfolgt werden. Diese Überwachungsmöglichkeit kann genutzt werden, um Grundlagenforschung zur Zelldifferenzierung zu betreiben. Darüber hinaus ergibt sich die Möglichkeit zur pharmakologischen Untersuchung neuer Substanzen, die auf die Differenzierung von Zellen wirken. Als dritte Option erschließt sich die Möglichkeit, mit der beschriebenen Erfindung die Qualität einer gewonnenen Stammzellprobe schnell und effizient zu überprüfen.

Bisher werden radiologische Untersuchungen, klassische histologische Untersuchungen und Immunhistochemie dazu genutzt, die zu erwartenden Eigenschaften eines Tumors zu ermitteln. Darüber hinaus werden vermehrt Genprofile erstellt, um Tumore zu klassifizieren. Die bekannten Verfahren ermöglichen zwar eine Klassifizierung des zu behandelnden Tumors, sind aber nachteilig für Studien, bei denen ein hoher Datendurchsatz bei gleichzeitiger Kostenminimierung von Bedeutung ist. Histologische Untersuchungen sind zwar durchaus in kurzen Zeiträumen mit Hilfe von Färbeautomaten durchführbar, in der Regel aber kostenintensiv. Die histologische Untersuchung erfordert immer die Fixierung und Färbung des zu untersuchenden Gewebes und ist daher zwangsläufig mit einem hohen Chemikalienbedarf verbunden. Bei immunhistochemischen Verfahren entstehen ebenfalls hohe Kosten aufgrund des Bedarfs an spezifischen Antikörpern. Gerade bei der Suche nach neuen pharmakologisch wirksamen Substanzen hat das High-Throughput-Screening besondere Bedeutung, da in möglichst kurzen Zeiträumen möglichst viele potentielle Wirkstoffe getestet werden sollen.

Die Vorrichtung und/oder das Verfahren können zumindest in besonderen Ausführungsformen dazu eingesetzt werden, heterogene Gewebeproben aus Tumorbiopsien auf ihre histologische Zusammensetzung zu überprüfen. Die daraus gewonnenen Daten können herangezogen werden, um den Entwicklungsgrad und das maligne Potenzial der getesteten Tumore detailliert beurteilen zu können.

Die Vorrichtung und/oder das Verfahren in zumindest besonderen Ausführungsformen dazu eingesetzt werden, neuentwickelte Wirkstoffe zur Behandlung maligner Tumore kosteneffizient und zeitsparend auf ihre Wirksamkeit zu prüfen. Die so gewonnenen Daten können dazu eingesetzt werden, das mögliche Einsatzfeld und die Effizienz eines potentiellen Therapeutikums zu ermitteln.

Das Verfahren und/oder die Vorrichtung können zumindest in besonderen Ausführungsformen im Zuge der Entwicklung individualisierter Behandlungsansätze für individuelle Krebspatienten dazu eingesetzt werden, Biopsie-Material aus malignen Tumoren auf seine Empfindlichkeit gegenüber der geplanten Behandlung mit ausgewählten Chemotherapeutika zu testen und somit die Wirksamkeit der geplanten Therapie vorab zu ermitteln. Die so gewonnenen Daten können dazu verwendet werden, die Effizienz der Chemotherapie zu optimieren bei gleichzeitiger Minimierung des Leidensdrucks der Patienten.

In einer besonderen Ausführungsform der vorliegenden Erfindung kann zum Beispiel ein Array, das aus Feldeffekttransistoren (FETs) besteht, zur impedimetrischen Analyse der Zelladhäsionsfähigkeit als Indikator für Zellviabilität genutzt werden. Dazu wird die Transistor- Transferfunktion (Übertragungsfunktion) gemessen. Unter der Übertragungsfunktion des Feldeffekttransistors wird hier insbesondere der Frequenzgang eines durch die Source-Drain-Strecke fließenden Stroms oder einer davon abgeleiteten Größe in Abhängigkeit der Frequenz der Wechselspannung verstanden. Beispielsweise kann ein Messinstrument zur Messung von Amplitude und Phase eines durch die Source-Drain-Strecke des Feldeffekttransistors fließenden Stroms vorgesehen sein, der hierzu vorzugsweise in einer ersten Verstärkerstufe mittels geeigneter Elektronik in eine Ausgangsspannung ($V_{out}$) gewandelt wird. Der Zeitverlauf dieser Größen enthält eine Faltung der Impedanz der Probe mit der Übertragungsfunktion des Feldeffekttransistors. Ist diese Übertragungsfunktion bekannt, beispielsweise aus einer Messung ohne Probe, lässt sich die Faltung invertieren und die Impedanz der Probe durch Entfaltung ermitteln. Die Impedanz ist in der Zellbiologie eine etablierte passive Messgröße, sodass die Möglichkeit der Vorrichtung, diese zu bestimmen, die Vergleichbarkeit der Messergebnisse mit den Ergebnissen anderer Versuche deutlich erhöht.

Wenn die einzelnen FETs eine maximale Größe von zum Beispiel $25 \times 5 \mu m^2$ aufweisen, so sind sie kleiner als einzelne tierische und damit auch menschliche Zellen. Die dadurch erreichte Auflösung erlaubt es, sowohl in konfluenten Kulturen als auch bei geringerer Zelldichte Aussagen über die Reaktionen auf potentiell zytotoxische Substanzen zu treffen. Die Reaktion der Zellen auf einen definierten Stimulus kann sowohl mittels eines Impedanzspektrums in einer Endpunktanalyse oder in Echtzeit unter Kulturbedingungen ermittelt werden.

Im Stand der Technik werden Gold-Elektroden in einer Größenordnung zwischen 25 und $250 \mu m$ im

Durchmesser, bzw. wesentlich größere Sensorenflächen, zur impedimetrischen Analyse der Zelladhäsion und Zellviabilität verwendet. Die oben genannten erfindungsgemäßen Verwendungen umfassen im Gegensatz dazu Silizium-basierte und noch bevorzugter organische Feldeffekttransistoren. Diese können deutlich kleiner hergestellt werden und ermöglichen dadurch eine viel höhere Auflösung als vergleichbare MetallElektroden-Systeme. Durch die höhere Auflösung können detaillierte Daten zur Reaktion heterogener Zellpopulation auf einen zu testenden Wirkstoff gewonnen werden.

Wie bereits oben ausgeführt wurde, kann/können die Vorrichtung und/oder das Verfahren zumindest in einer besonderen Ausführungsform zur histologischen Analyse von Tumorbiopsie-Proben und damit zur schnellen Analyse einer Tumorzusammensetzung eingesetzt werden. Diese Proben sind heterogener Natur und reagieren daher nicht zwangsläufig uniform auf die Behandlung mit einem spezifischen Wirkstoff. Daher erweisen sich die bisher kommerziell verfügbaren Messtechniken als nur bedingt effektiv, da eine größere Zellpopulation nicht in ihre einzelnen Bestandteile aufgelöst werden kann.

Zur Vorabprüfung der Wirksamkeit verabreichter Chemotherapeutika wird Biopsie-Material dissoziiert und auf einer Messfläche einer Vorrichtung gemäß einer besonderen Ausführungsform der vorliegenden Erfindung kultiviert. Anschließend können die so erzeugten Proben auf ihre Reaktion auf einen eingesetzten Wirkstoff untersucht werden und so einen Eindruck über die zu erwartende Effizienz einer geplanten Chemotherapie geben. Durch diese Vorabuntersuchung der Wirksamkeit einer geplanten Therapie können mögliche Nebenwirkungen bei einer ineffizienten oder vollständig unwirksamen Therapie verringert oder vollständig vermieden werden. Wie ebenfalls oben ausgeführt wurde, ermöglicht die vorliegende Erfindung zumindest in einer besonderen Ausführungsform die Etablierung neuer Hochdurchsatz-Verfahren zur Wirkstoffanalyse. Diese kann zeitsparend und kosteneffizient im Hinblick auf ihre generelle Wirksamkeit, Spezifizität und mögliche unerwünschte zytotoxische Effekte durchgeführt werden. Im Gegensatz zu bereits kommerziellen Systemen zur impedimetrischen Zellanalyse können durch die vorliegende Erfindung pharmakologische Untersuchungen auf der Auflösungsebene einer einzelnen Zelle durchgeführt werden. Darüber hinaus kann durch die Verwendung mehrerer Sensorchips eine große Anzahl an Proben einfach und schnell untersucht werden, was eine Etablierung neuer Hochdurchsatz-Verfahren zur Wirksamkeitsanalyse neuer Wirkstoffe in komplexen Zellsystemen oder in etablierten Zelllinien ermöglicht.

Auch wenn oben die Rede von Einweg-Sensoren war, können die Sensoren (Transistorchips) auch wiederverwertet werden. Darüber hinaus kommen die Vorrichtungen vollständig ohne Einsatz von Färbechemikalien aus. Dadurch sind die Kosten für Verbrauchsmaterialen vergleichsweise gering.

Wie sich anhand der obigen Ausführungen ergibt, ist die Erfindung für breite Anwendungen einsetzbar.
In der nachfolgenden Beschreibung, werden mehrere Ausführungsbeispiele anhand der schematischen Zeichnungen im Einzelnen erläutert. Dabei zeigt:

Figur 1      eine Draufsicht auf (links) und eine Schnittansicht entlang der gestrichelten Linie (rechts) einen/eines organischen Feldeffekttransistor(s) einer Vorrichtung zur Messung biologischer und/oder elektronischer Eigenschaften einer Probe gemäß einer besonderen Ausführungsform der vorliegenden Erfindung;

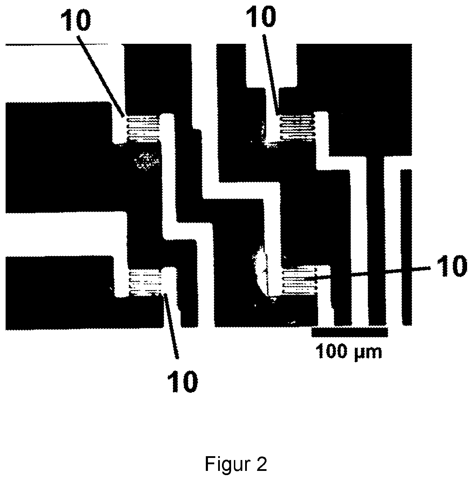

Figur 2      eine Mikroskopieaufnahme eines Sensor-Chips gemäß einer besonderen Ausführungsform der vorliegenden Erfindung mit vier organischen Feldeffekttransistoren (OFETs);

Figur 3      eine Ausgangskennlinie eines organischen Feldeffekttransistors gemäß einer weiteren besonderen Ausführungsform der Vorrichtung zur Messung biologischer und/oder elektronische Eigenschaften einer Probe, gemessen in flüssiger Umgebung (links) und eine Transferkennlinie desselben organischen Feldeffekttransistors (rechts);

Figur 4      das Ergebnis der Messung eines Leckstromes durch eine Gate-Elektrode während des Betriebs des organischen Feldeffekttransistors von Figur 3;

Figur 5      das Ergebnis der Messung des Signalübertrags (Bandbreite) des organischen Feldeffekttransistors von Figur 3 und die hypothetische Verschiebung der Bandbreite durch Verkleinerung der Gate-Länge L;

Figur 6      ein Ersatzschaltbild zur Simulation des Signalübertrags eines organischen Feldeffekttransistors und einer ersten Vorverstärkerstufe, wenn eine Zelle auf dem Feldeffekttransistor bzw. dem Sensor angewachsen ist;

Figur 7      eine schematische Darstellung zur Erklärung des Dämpfungseffekts von Zellen auf OFETs mit dem Material PEDOT:PSS.

Figur 8      das Ergebnis der Simulation des Adhäsionseffekts einer einzelnen Zelle auf einem organischen Feldeffekttransistor auf den Signalübertrag unter Nutzung des Ersatzschaltbildes in Figur 6;

Figur 9    das Ergebnis einer realen Messung des Adhäsionseffekts von mehreren HL-1-Zellen auf einem OFET-Sensor; und

Figur 10    das Ergebnis einer realen Messung des Adhäsionseffekts einer einzelnen HL-1-Zelle auf einem kleinen OFET-Sensor.

Wie bereits oben ausgeführt wurde, können durch eine hohe Integrationsdichte vieler FET-Sensoren in einer Zellkulturschale mehr Daten - auch von verschiedenen Gewebearten - vermessen werden, als es momentan mit den auf dem Markt befindlichen Systemen möglich ist. Dazu muss die Größe eines FET-Sensorpunktes (Messfläche) kleiner als zum Beispiel 20x20 $\mu m^2$ sein, was in etwa die obere Grenze des Durchmessers einer einzelnen Zelle ist. Die kleinsten für das ECIS-System erhältlichen Elektroden haben einen Durchmesser von 25 $\mu m$. Bisher genutzte Silizium-basierte FET-Sensoren haben typische Größen im Bereich von 10 $\mu m$. Dabei ist wesentlich, dass bisher die halbleitenden Polymere eine geringere Ladungsträgerbeweglichkeit im Vergleich zu Silizium (0,1 $cm^2$/Vs für Ladungsträger im Material PEDOT:PSS - bis zu 3,6 $cm^2$/Vs bei Nutzung von Ethyleneglycol - im Vergleich zu 1400 $cm^2$/Vs für Elektronen in Silizium (Semiconductor Physics and Devices: Basic Principles, Donald A. Neamen, McGraw-Hill Publ. Comp. 3. Auflage, Internationale Edition, 2002)) aufweisen. Dadurch ist in der Regel die Polymerelektronik zu langsam und eine Aufnahme eines Spektrums in dem Bereich wie in der WO 208/061489 A1 ausgeschlossen. Das Erreichen von Frequenzbereichen über 10 kHz ist jedoch zur Ausnutzung der Impedanzmethode zur Messung der Zelladhäsion wichtig, wenn nicht sogar essentiell.

Die Grenzfrequenz einer FET-Struktur kann in der erster Näherung beschrieben werden durch folgende Formel (Semiconductor Physics and Devices: Basic Principles, Donald A. Neamen, McGraw-Hill Publ. Comp. 3. Auflage, Internationale Edition, 2002):

$$f_T = \frac{g}{2\pi x C_G} = \frac{\mu_n (V_{GS} - V_T)}{2\pi x L^2},$$

mit $f_T$:Grenzfrequenz, $g_m$:Transconductance [mS], $\mu_m$: Ladungsträgerbeweglichkeit [$cm^2$/Vs], $V_{GS}$:Spannung zwischen Gate- und Source-Kontakt des Transistors [V], $V_T$: Anlaufspannung des Transistors [V], L:Gate-Länge des Transistors [m], $C_G$: Kapazität der Isolatorschicht auf der Gate-Elektrode.

Dabei sind aufgrund der wesentlich geringere Ladungsträgerbeweglichkeiten der organischen Halbleitermaterialien mit OFETs ca. 5 Größenordnungen kleinere Grenzfrequenzen zu erwarten als mit Silizium-basierten FETs. Wie bereits in der WO 2008/061489 A1 ausgeführt ist, ist für den Feldeffekttransistor mit einer adhärierten Zelle ein signifikant unterschiedlicher Verlauf der Übertragungsfunktion festzustellen. Grundsätzlich führt die Zellabdeckung des Feldeffekttransistors zu einer unterschiedlichen Übertragungsfunktion des Feldeffekttransistors. In allen Fällen nimmt der Feldeffekttransistor schon bei wesentlich geringeren Frequenzen nicht mehr an der Gesamtsignalverstärkung des Systems teil und wird die angelegte Wechselspannung nicht mehr verstärkt (hier: für Frequenzen oberhalb von $f_{sin}$=10 kHz).

Die Erweiterung des Frequenzbereichs der OFET-Sensoren kann im Wesentlichen durch einen oder mehrere der nachfolgend beschriebenen Maßnahmen erreicht werden:

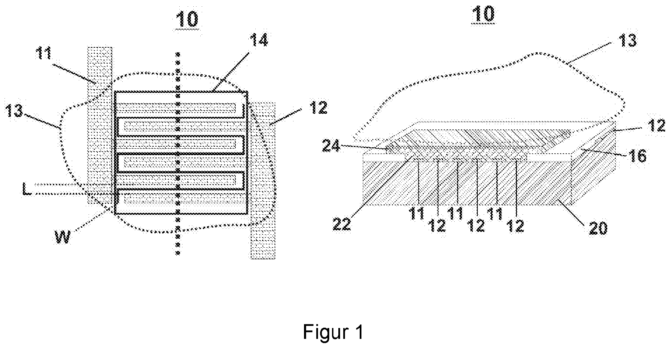

1. Geeignete Dimensionierung, so dass das Verhältnis Gate-Breite W/Gate -Länge L der FET-Struktur gesteigert wird (wie in Figur 1 gezeigt).
Bei dem OFET 10 von Figur 1 wird eine sogenannte interdigitierende Fingerstruktur (IDE) verwendet, um den zur Verfügung stehenden Platz (Messfläche) (20x20 $\mu m^2$) maximal auszunutzen, unter Minimierung von Gate-Länge L, wobei L der Abstand zwischen den einzelnen Fingern der Source und des Drains im Querschnitt ist (siehe auch Figur 1 rechts). Die IDE-Struktur weist einen Source-Kontakt 10 und einen Drain-Kontakt 12 auf. Mit 14 ist die Öffnung eines Gate-Bereiches in einer Passivierungsschicht 16 gezeigt. Eine adhärierte Zelle 13 ist mit gestrichelten Linien angedeutet.
Ein weiteres Beispiel wären zwei Spiralen, die ineinandergeschlungen sind, oder vergleichbare Geometrien.
Wie sich anhand des Querschnitts entlang der gestrichelten Linie in Figur 1 links in der Figur 1 rechts ergibt, sind die Source-Kontakt 11-Finger und Drain-Kontakt 12-Finger auf einem Substrat 20 angeordnet. Das Substrat 20 ist optisch transparent. Es kann zum Beispiel aus Glas, Polymermaterial, z.B. Capton, Neopulim® oder ähnlichem, hergestellt sein. Die Zuleitungen 18 zu den in der Figur gezeigten Source- und Drain-Kontakten bspw. aus Metall, z.B. Gold, oder aus einem transparenten leitfähigen Material, wie z.B. Indium-Zinn-Oxid (ITO) sind ebenfalls von besagter Passivierungsschicht 16 so bedeckt, dass die Source- und Drainkontakte 11 und 12 gegenüber einer Flüssigkeit, wie z.B. Zellkuturflüssigkeit, isoliert sind. Die Passivierungsschicht 16 kann zum Beispiel aus $SiO_2$, $Si_3N_4$ oder bspw. auch Parylene bestehen. Über den Source-Kontakt 11- und Drain-Kontakt 12-Fingern ist ein elektrisch leitfähiges, optisch transparentes Halbleitermaterial 22, wie z.B. PEDOT: PSS, aber auch bspw. Graphen, angeordnet. In dem Halbleitermaterial 22 wird der Kanal des OFET 10 gebildet. Das Halbleitermaterial 22 ist wiederum mit einer Isolierschicht 24 z.B. aus Ethylenglycol versehen.
Es ist ebenfalls bekannt, dass die Geschwindigkeit eines PEDOT:PSS-OFETs sich durch eine möglichst dünn ausgeprägte Schicht an Polymermaterial steigern lässt. Daher sollte vorteilhafterweise ein Herstellungsverfahren mit möglichst dünner Auftragung des Polymermaterials (wie z.B. Inkjetprinting oder Spincoating) genutzt werden.

2. Passivierung des OFETs 10 bzw. des Sensors bzw. des Sensor-Chips durch eine isolierende Schicht (Passivierungsschicht 16) bspw. aus Siliziumdioxid oder Siliziumnitrid, wobei eine Öffnung nur der relevanten IDE-Struktur zum Kontakt mit dem Halbleitermaterial 22 erfolgt (siehe Figur 1).

Allgemein werden dadurch die Reduktion des Einflusses der parasitären Eigenschaften von Zuleitungen und die Minimierung von Leckströmen angestrebt. Dazu werden elektrisch leitfähige Zuleitungsmaterialien (am besten auch optisch transparent, wie z.B. Indium-Zinn-Oxid (ITO)) oder auch elektrisch leitfähige Polymere verwendet. Die Passivierung der Zuleitungen erfolgt bzw. die Passivierungsschicht 16 wird mit geeigneten Materialien in geeigneter Dicke erzeugt, um die parasitäre Kapazität der Zuleitungen geeignet einzustellen.

3. Nutzung eines geeigneten Halbleitermaterials 22 im OFET-Kanal, vorzugsweise eines halbleitenden Polymermaterials, wie z.B. PEDOT: PSS, oder Materialien wie Graphen, bzw. reduziertes Graphenoxid oder ähnliche 2D-Materialien (Chalcogenide, wie z.B. $MoS_2$), die vorzugsweise günstig und druckbar sind, bzw. auch Kombinationen und Mischungen der genannten Materialien. Generell sollten diese Materialien eine hohe Ladungsträgerbeweglichkeit $\mu$ zeigen. Bei PEDOT: PSS (Poly-3,4-ethylendioxydthiophen) liegt die typischer Ladungsträgerbeweglichkeit in der Größenordnung von 0,1 cm$^2$/Vs (bis zu 3,6 cm$^2$/Vs wie oben beschrieben) und ist daher relativ hoch für diese Materialklasse.

4. Nutzung eines geeigneten Materials (Isolationsschicht 24) zur Stabilisierung der Gatestruktur, wie z.B. Ethylenglycol. Es können aber auch im Falle der 2D-Materialien andere Isolatoren (in Bezug auf Leckströme im dc-Bereich) mit hoher relativer Dielektrizitätszahl $\varepsilon_r$ abgeschieden werden (z.B. durch Atomic Layer Deposition ALD).

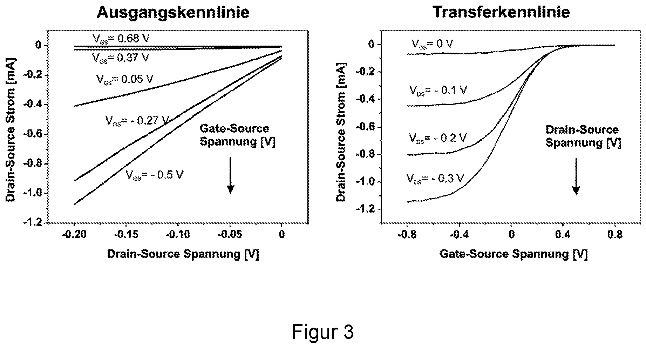

Zur Demonstration der Methode und des Verfahrens wurde von den hiesigen Erfindern eine IDE Struktur aus Gold auf einem Glassubstrat mit klassischen lithographischen Methoden erzeugt. Die Zuleitungen wurden durch SiO$_2$ isoliert und der Gatebereich mit der IDE-Struktur im nachfolgenden Schritt wieder freigelegt. Das genutzte Halbleitermaterial war PEDOT:PSS und die Isolation des Materials wurde durch Ethylenglycol realisiert. In Figur 3 links ist dazu die Ausgangskennlinie des von den hiesigen Erfindern hergestellten OFETs, gemessen in flüssiger Umgebung, gezeigt. Genauer gesagt ist der Strom I_D in Abhängigkeit von der Spannung V_DS für verschiedene V_GS dargestellt. In der Figur 3 rechts ist die Transferkennlinie des gleichen OFETs dargestellt. Genauer gesagt ist der Strom I_D in Abhängigkeit von der Spannung V_GS für verschiedene V_DS dargestellt. An beiden Kennlinien kann man ein sehr gutes Transistorverhalten erkennen. Sehr vorteilhaft ist, dass der OFET schon bei einer Spannung um 0 V_GS eine gute Verstärkung zeigt, was sehr vorteilhaft zum Betrieb in Flüssigkeiten und zur Verwendung mit Zellkulturen ist, da hier mit sehr geringen Spannungen gearbeitet werden kann. Insgesamt zeigt die Figur 3, dass die hiesigen Erfinder erfolgreich OFETs zur Nutzung in Flüssigkeiten hergestellt haben.

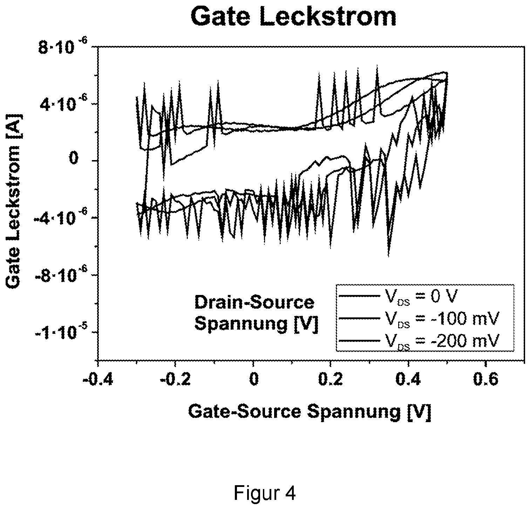

In der Figur 4 ist zu sehen, dass die Isolation mit Ethylenglycol hervorragend funktioniert und keine Leckströme durch die Gate-Elektrode fließen. Genauer gesagt zeigt die Figur 4 den gemessenen Leckstrom I_G durch eine Gate-Elektrode in Abhängigkeit von der Spannung V_GS während des Betriebs des OFETs für verschiedene V_DS. Die Messergebnisse zeigen die effektive Isolation und Stabilisierung durch die Nutzung von Ethylenglycol. Der gemessene Leckstrom ist durch die isolierende Wirkung von Ethylenglycol um 3 Größenordnungen kleiner als der gemessene Drain-Source-Strom in Fig. 3. Damit ist eine sehr gute Funktion des OFETs in der Flüssigkeit gewährleistet. Im Vergleich zu den Strömen von 1 mA (siehe Figur 3) sind die Leckströme im Bereich von $\mu$A zu vernachlässigen. Folglich ist eine stabile Arbeitsweise der OFETs in einer Zellkultur gewährleistet. Damit ist die Voraussetzung geschaffen, die Zellimpendanzmethode, wie sie in der WO 2008/061489 A1 beschrieben ist, nutzen zu können.

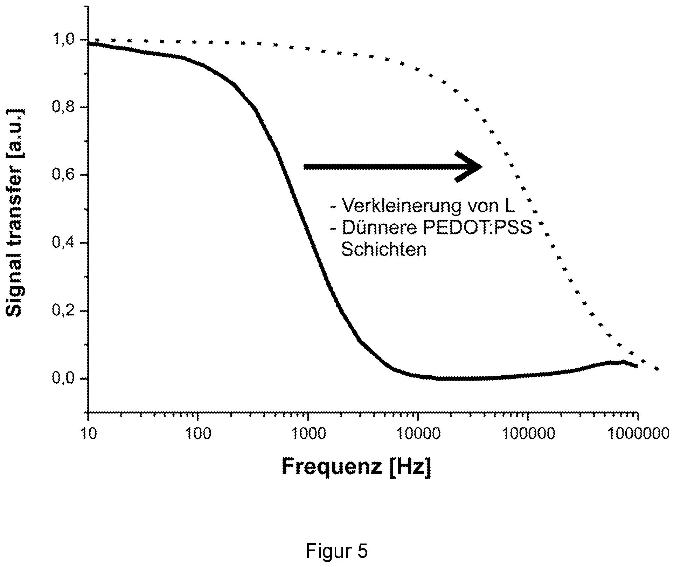

Durch die Nutzung von Ethylenglycol in Kombination mit PEDOT:PSS erhöht sich auch die Ladungsträgerbeweglichkeit auf 3,6 cm$^2$/Vs (M. Yamashita, C. Otani, M. Shimizu, and H. Okuzaki, Effect of solvent on carrier transport in poly(3,4-ethylenedioxythiophene)/poly(4-styrenesulfonate) studied by terahertz and infraredultraviolet spectroscopy, Appl. Phys. Lett. 99, 143307 (2011)), was im Frequenzbereich etwa eine Größenordnung bringt. Bei dem momentan vorhandenen Design der OFET-Sensoren beträgt die Gate-Länge L ca. 5 $\mu$m. Derartige OFET-Sensoren erreichen bereits Frequenzen bis zu 1 kHz (siehe Figur 5). Figur 5 zeigt das Ergebnis der Messung des Signalübertrags (Bandbreite) des OFETs (Daten normalisiert auf den 10 Hz-Wert). Der 3 dB-Punkt befindet sich bei 440 Hz für den ausgewählten OFET. Die gestrichelte Linie zeigt den zu erwartenden Effekt, wenn die Gate-Länge L von momentan 5 $\mu$m auf 200 nm verkleinert wird (nach der oben angegebenen Formel).

5. Verringerung der Gate-Länge L auch über die typische optische Auflösungsgrenze (ca. 1 $\mu$ m) hin-

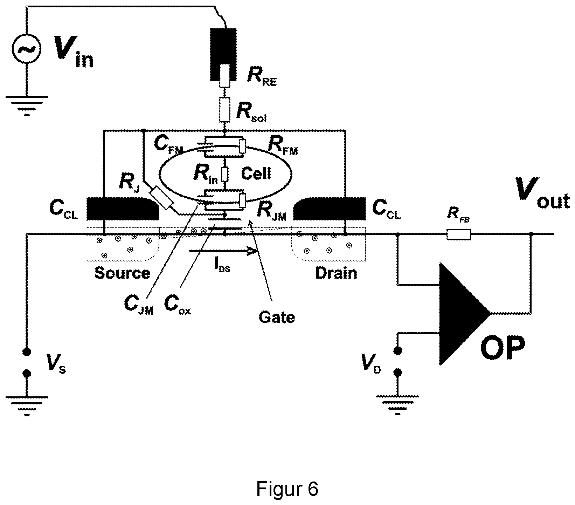

aus. Nach der oben angegebenen Formel ist eine quadratische Abhängigkeit zu erwarten. Mittels bspw. einer Nanoimprint-Lithographieanlage ist eine Skalierung in Bereiche um L=200 nm realistisch. Zur Simulation des Frequenzgangs der Konfigurationszelle auf OFET kann ein Ersatzschaltbild verwendet werden, wie es beispielhaft in Figur 6 gezeigt ist. Die in Figur 6 gezeigten Parameter haben folgende Bedeutungen:

$v_{in}$: Eingangssignal

$v_{out}$: Ausgangssignal

$R_{RE}$: Widerstand der Referenzelektrode plus Widerstand der Flüssigkeit zwischen Referenzelektrode und OFET

$V_S$ und $V_D$: Spannungen an Source- und Drainkontakten

OP: Operationsverstärker des Transimpedanzverstärkers

$R_{FB}$: Rückkopplungswiderstand des Transimpedanzverstärkers

Source, Drain, Gate: Drei Kontakte des OFETs

$I_{DS}$: Drain-Source Strom durch den Kanal des OFETs

$C_{ox}$: Kapazität des Gatekontakts, die sich aus dem isolierenden Material auf dem Gate bildet.

$C_{CL}$: Kapazitäten der Drain- und Sourcezuleitungen.

$R_{in}$: Innenwiderstand der Zelle

$R_J$: Abdichtwiderstand der Zelle - Parameter zu Beurteilung der Zelladhäsion

$C_{FM}$ und $R_{FM}$: Kapazität und Widerstand der freien Zellmembran - Parameter zur Beurteilung der Zellvitalität

$C_{JM}$ und $R_{JM}$: Kapazität und Widerstand der adhärierten Zellmembran - Parameter zur Beurteilung der Zellvitalität

Passive Eigenschaften bzw. Messgrößen der Zellen sind $C_{FM}$, $R_{FM}$, $R_{in}$, $C_{JM}$ und $R_J$. Aus der Analyse dieser Messgrößen erhält man Informationen über z. B. die Adhäsionsstärke die Zellform und die Integrität der Zellmembranen ("Gesundheitszustand" der Zellen bzw. Entwicklungsstatus der Zellen).

Das Ersatzschaltbild dient zur Simulation des Signalübertrags eines OFETs und einer ersten Vorverstärkerstufe, wenn eine Zelle auf dem OFET angewachsen ist. Im wesentlichen dämpft die Zelle den Signalübertrag im höherfrequenten Bereich. Aus den Spektren kann man - bei bekannten Parametern des OFET-Chips inklusive der parasitären Effekte der Zuleitungen und des Frequenzgangs des Verstärkers - die relevanten Zellparameter wie Abdichtwiderstand ($R_J$) und Membrankapazität ($C_M$) herausfitten. Die zu erwartenden Spektren zur Adhäsion einzelner Zellen können mit dem in Figur 6 gezeigten Ersatzschaltbild simuliert werden.

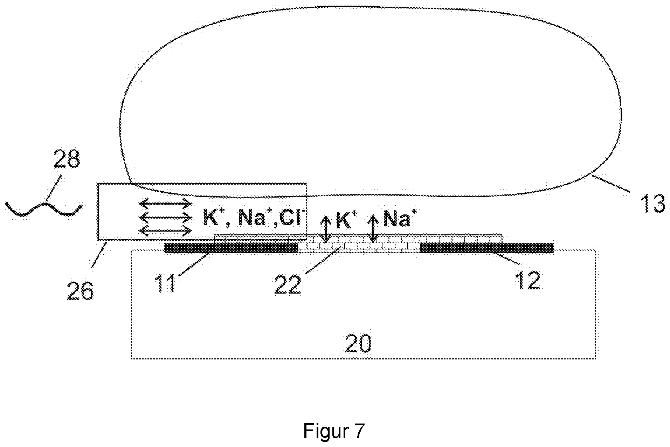

Der durch die Zelle erzeugte Dämpfungseffekt in den höheren Frequenzen kann folgendermaßen erklärt werden: In Figur 7 ist schematisch die Zelle adhäriert auf einer vereinfachten OFET-Struktur gezeigt. Das Stimulationssignal in der Flüssigkeit wird durch Ionen in der Flüssigkeit - vornehmlich Natrium, Kalium und Chlorid im Falle von Nervenzellen - getragen. Das Material PEDOT:PSS tauscht im Wesentlichen Kationen - in diesem Beispiel Natrium und Kalium - mit seiner flüssigen Umgebung aus. Diese Ionen binden an die Polymerketten des Materials und modulieren die elektrische Leitfähigkeit im Material. Wenn das OFET Gate frei zugänglich ist, dann geschieht das mit einer gewissen Effektivität und maximaler Geschwindigkeit. Wenn jetzt eine Zelle auf dem Sensor angewachsen ist, dann bildet sich ein elektrochemischer Abdichtwiderstand $R_J$ wie z.B. aus Pabst et al., 2007 (M. Pabst, G. Wrobel, S. Ingebrandt, F. Sommerhage, and A. Offenhäusser, Solution of the Poisson-Nernst-Planck equations in the cell-substrate interface, European Physical Journal E - Soft Matter 24, 1-8, (2007)) bekannt. Dieser Widerstand behindert die freie Diffusion der Ionen im dünnen Spalt zwischen Zellmembran und OFET (typischerweise im Bereich von 50 nm) und führt dadurch zu einer Dämpfung, die sich im Impedanzspektrum bemerkbar macht.

Das Signal koppelt über Austausch von Natrium- und Kaliumionen mit der Flüssigkeit in das Polymermaterial ein. Durch die enge Adhäsion der Zelle ist die Diffusion von diesen Ionen an das Transistorgate behindert, was zu einer Dämpfung im Impedanzspektrum führt.

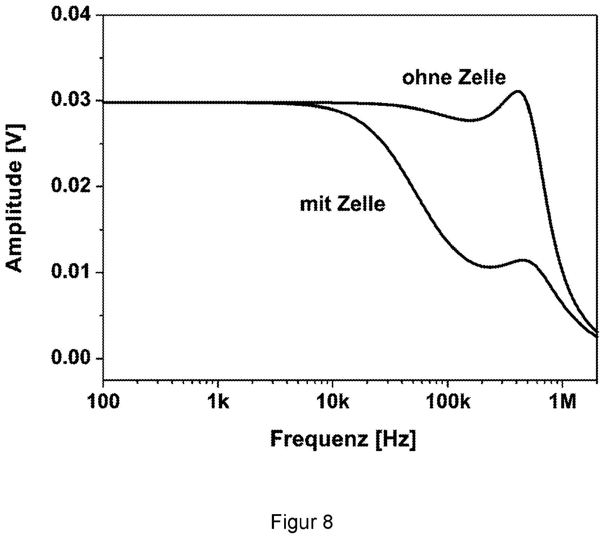

In Figur 8 ist eine exemplarische Simulation gezeigt. Die Effekte sind sehr vergleichbar zu den bereits mit den früheren Silizium-basierten FET-Sensoren gemessenen.

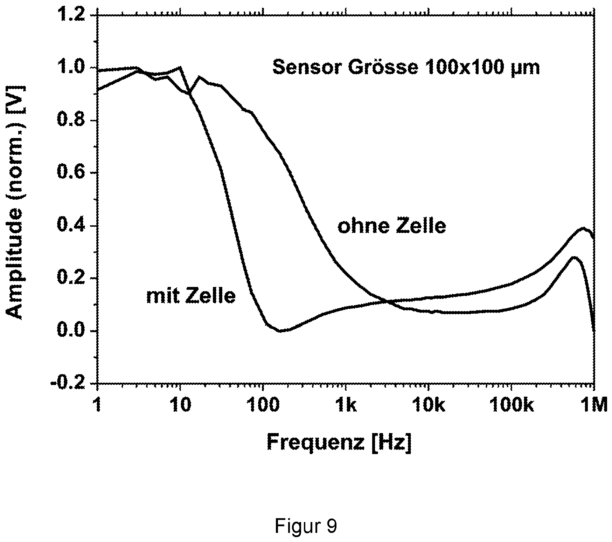

In Figur 9 ist das Ergebnis einer realen Messung des Adhäsionseffekts von HL-1-Zellen auf einem OFET-Sensor (Sensorpunkt bzw. Messfläche 100x100 $\mu m^2$) gezeigt. Aufgrund der relativ großen Messfläche entspricht diese der Fläche von 2-4 Zellen aus einem Zellrasen.

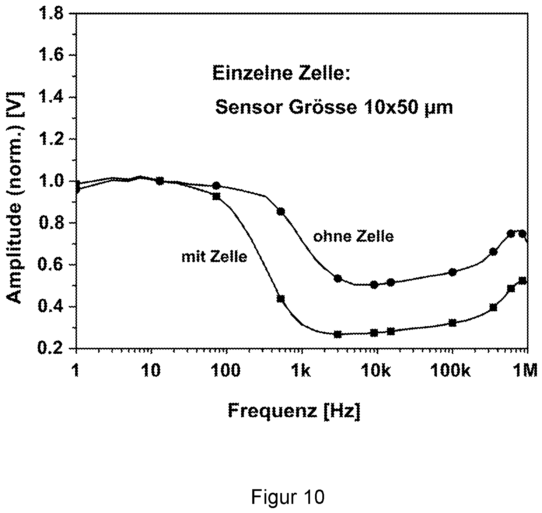

Schließlich ist in Figur 10 das Ergebnis einer Messung des Adhäsionseffekts einer HL-1-Zelle auf einem kleineren OFET-Sensor gezeigt. Die kleinere Messfläche ermöglicht eine echte Einzelzellauflösung der Methode auf den transparenten, günstigen Einweg-OFET-Sensoren. Da der genutzte Gate-Abstand 5 μm beträgt, spielt sich alles noch bei niedrigeren Frequenzen als in der Figur 8 ab. Es ist bei etwa 100 Hz ein klarer Unterschied zu sehen.

Die Methode ist auf verschiedenen Zelltypen von unterschiedlichen Quellen anwendbar. Dazu zählen Zellen von Versuchstieren (Ratten, Mäuse etc.), Stammzellen, pflanzliche Zellen, menschliche Zellen aus primärem Gewebe (z.B. Biopsien) sowie Zellen von Zelllinien. Die obengenannten, kommerziellen Systeme müssen in der Regel mit Zellen genutzt werden, die geschlossene Schichten bilden (Epitel(Haut)Zellen, Zellen von Hauttumoren, usw.).

Im Vergleich zu den auf dem Markt befindlichen Systemen kann man die Methode sicherlich auch wesentlich höher integrieren, d.h. mehr Sensorpunkte auf kleinerem Raum unterbringen. Somit wird die gesamte Zellkultur untersucht und es können statistische Einzelzelldaten erhoben werden. Es würde z.B. eine Zellkultur aus einer Biopsie eines Tumors eines Patienten vollständig untersucht werden können. Typischerweise bestehen Tumore nicht nur aus einem Zelltyp, sondern aus verschiedenen einzelnen Zelltypen. Falls die Zelltypen in ihren individuellen Adhäsionsspektren unterscheidbar sind (erste Messungen lassen den Schluss zu), dann kann z.B. die Wirkung von Anti-Tumormedikamenten auf die unterschiedlichen Zelltypen eines Tumors individuell untersucht werden.

Die vorgestellte Methode lässt einen breiten Einsatz zu und die in der Erfindung erzielte radikale Reduktion der Herstellungskosten könnte zu sehr wirtschaftlichen Einweg-Sensoren führen.

BEZUGSZEICHENLISTE

10　　OFET
11　　Source-Kontakt
12　　Drain-Kontakt
13　　Zelle
14　　Gate-Bereich
16　　Passivierungsschicht
20　　Substrat
22　　Halbleitermaterial
24　　Isolierschicht
26　　Abdichtwiderstand $R_J$
28　　Stimulationssignal

**Patentansprüche**

1. Vorrichtung zur Messung biologischer und/oder elektronischer Eigenschaften einer Probe, insbesondere zur Messung von Zellparametern insbesondere einzelner menschlicher, pflanzlicher oder tierischer Zellen, umfassend einen Feldeffekttransistor mit einer Kontaktierungseinrichtung zur Kontaktierung der Probe mit dem Gate des Feldeffekttransistors, eine Stimulationseinrichtung zur Beaufschlagung der Probe mit einer elektrischen Wechselspannung und ein frequenzselektives Messinstrument zur Messung von Amplitude und Phase eines durch die Source-Drain-Strecke des Feldeffekttransistors fließenden Stroms,

   wobei der Feldeffekttransistor ein organischer Feldeffekttransistor (10) ist und das organische Material des Feldeffekttransistors eine Ladungsträgerbeweglichkeit von 0,1cm$^2$/Vs bis 3,6 cm$^2$/Vs aufweist.

2. Vorrichtung nach Anspruch 1, **dadurch gekennzeichnet, dass** der Feldeffekttransistor ein Gate-Breite W/Gate-Länge L-Verhältnis im Bereich von 1 bis 10000, vorzugsweise im Bereich von 10 bis 1000 aufweist und besonders bevorzugt 100 beträgt und / oder der Feldeffekttransistor eine Fingerstruktur oder eine spiralförmige Struktur aufweist.

3. Vorrichtung nach einem der vorangehenden Ansprüche, **dadurch gekennzeichnet, dass** die Messfläche des Feldeffekttransistors kleiner als ca. 400 μm$^2$ ist und/oder der Durchmesser der Messfläche kleiner als ca. 28 μm ist und/oder die gesamte Messfläche oder nur ein Teil derselben optisch transparent ist.

4. Vorrichtung nach einem der vorangehenden Ansprüche, **dadurch gekennzeichnet, dass** die Gate-Länge L kleiner als ca. 1 μm ist und / oder der Feldeffekttransistor mittels eines Nanostrukturierungsverfahrens, wie z. B. Elektronenstrahllithografie oder deep-UV-Lithographie oder Nanoimprintlithografie, strukturiert ist.

5. Vorrichtung nach einem der vorangehenden Ansprüche, **dadurch gekennzeichnet, dass** die Source- und Drain-Zuleitungen aus einem elektrisch leitfähigen, vorzugsweise optisch transparenten Material oder einer entsprechenden Materialmischung bestehen und mit einer Passivierungsschicht, wie z. B. aus SiO$_2$, Si$_3$N$_4$ oder Parylene, versehen, insbesondere elektrisch isoliert, sind.

6. Vorrichtung nach einem der vorangehenden Ansprüche, **dadurch gekennzeichnet, dass** das organische Halbleitermaterial (22) des Feldeffekttransistors ein optisch transparentes Polymer, wie z.B. PEDOT:PSS, ein Oligomer oder ein 2D-Material, wie z. B. Graphen oder reduziertes Graphenoxid, Chalcogenid, wie z. B. MoS$_2$, oder eine Kombination oder Mischung von mehreren derselben ist.

7. Vorrichtung nach einem der vorangehenden Ansprüche, **dadurch gekennzeichnet, dass** das organische Halbleitermaterial (22) zur elektrischen Isolierung des Gates mit einer Isolierschicht (24), wie z. B. Ethylenglycol, überzogen ist.

8.	Vorrichtung nach einem der vorangehenden Ansprüche, **dadurch gekennzeichnet, dass** sie ein Substrat für den Feldeffekttransistor aus einem vorzugsweise optisch transparenten Material, wie z. B. Glas oder vorzugsweise optisch transparentes Polymermaterial, wie z. B. Kapton, Neopulim® oder ähnliches, oder aus einer Kombination derselben aufweist.

9.	Vorrichtung nach einem der vorangehenden Ansprüche, **dadurch gekennzeichnet, dass** sie als ein Sensor-Chip oder als ein Bestandteil desselben ausgebildet ist.

10.	Vorrichtung nach einem der vorangehenden Ansprüche, **gekennzeichnet durch** ein Anregungsmittel zur Beaufschlagung der Probe mit einer weiteren elektrischen Spannung oder einem elektrischen Strom.

11.	Array aus mindestens zwei Vorrichtungen nach einem der vorangehenden Ansprüche.

12.	Sensor-Chip mit mindestens einer Vorrichtung nach einem der Ansprüche 1 bis 10.

13.	Verfahren zur Messung biologischer und/oder elektronischer Eigenschaften einer Probe, insbesondere zur Messung von Zellparametern insbesondere einzelner menschlicher, pflanzlicher und tierischer Zellen, umfassend die Schritte:

	- Anordnen einer Probe in Kontakt mit dem Gate eines organischen Feldeffekttransistors einer Vorrichtung nach einem der Ansprüche der 1 bis 10,
	- Beaufschlagen der Probe mit einer Wechselspannung oder einem Wechselstrom und
	- Messen von Amplitude und Phase eines durch die Source-Drain-Strecke des Feldeffekttransistors fließenden Stroms.

14.	Verfahren nach Anspruch 13, **dadurch gekennzeichnet, dass** die Wechselspannung eine Frequenz zwischen 0,1 Hz und 1GHz, insbesondere zwischen 1Hz und 10MHz, aufweist und/oder die Wechselspannung eine Amplitude zwischen 0,001mV und 10V, insbesondere zwischen 1mV und 100mV, aufweist.

15.	Verfahren nach einem der Ansprüche 13 oder 14, **dadurch gekennzeichnet, dass** es orts- und/oder zeitaufgelöst durchgeführt wird.

16.	Verwendung der Vorrichtung nach einem der Ansprüche 1 bis 10, des Arrays nach Anspruch 11 oder des Sensor-Chips nach Anspruch 12 zur impedimetrischen Analyse insbesondere im Hochdurchsatz- und/oder Einzelzellverfahren adhärenter Zellen in vitro.

17.	Verwendung der Vorrichtung nach einem der Ansprüche 1 bis 10, des Arrays nach Anspruch 11 oder des Sensor-Chips nach Anspruch 12 zur elektronischen Analyse, insbesondere impedimetrischen Analyse, insbesondere im Hochdurchsatzverfahren von Stammzellendifferenzierung.

18.	Verwendung der Vorrichtung nach einem der Ansprüche 1 bis 10, des Arrays nach Anspruch 11 oder des Sensor-Chips nach Anspruch 12 zur elektronischen Biopsie insbesondere im Hochdurchsatzverfahren von menschlichen, pflanzlichen oder tierischen Geweben, insbesondere Tumorgewebe.

19.	Verwendung der Vorrichtung nach einem der Ansprüche 1 bis 10, des Arrays nach Anspruch 11 und des Sensor-Chips nach Anspruch 12 zur Analyse der Wirkungsweise von pharmazeutischen Substanzen und Medikamenten, insbesondere Krebsmedikamenten, wie zum Beispiel Zytostatika und Chemotherapeutika, oder Substanzen, die die Differenzierung von Stammzellen beeinflussen.

20.	Verwendung eines Verfahrens nach einem der Ansprüche 13 bis 15 zur elektronischen Analyse, insbesondere impedimetrischen Analyse, insbesondere im Hochdurchsatzverfahren von Stammzellendifferenzierung.

21.	Verwendung eines Verfahrens nach einem der Ansprüche 13 bis 15 zur elektronischen Biopsie insbesondere im Hochdurchsatzverfahren von menschlichen, pflanzlichen oder tierischen Geweben, insbesondere Tumorgewebe.

22.	Verwendung eines Verfahrens nach einem der Ansprüche 13 bis 15 zur Analyse der Zytotoxizität von Substanzen und Medikamenten, insbesondere Krebsmedikamenten, wie zum Beispiel Zytostatika und Chemotherapeutika.

23.	Verwendung eines Verfahrens nach einem der Ansprüche 13 bis 15 in Kombination mit einem Durchlichtmessverfahren, wie zum Beispiel einem Durchlichtmiröskopieverfahren.

24.	Zell-Chip-Hybridsystem zur Überwachung der Aktivität von menschlichen, pflanzlichen und tierischen Zellen und zur Analyse der Zytotoxizität von Substanzen und Medikamenten, insbesondere Krebsmedikamenten, wie zum Beispiel Zytostatika, und zur elektronischen Biopsie insbesondere im Hochdurchsatzverfahren von menschlichen und tierischen Geweben, insbesondere Tumorgewebe, und zur elektronischen Analyse, insbesondere impedimetrischen Analyse, insbesondere im Hochdurchsatzverfahren von Stammzellendifferenzierung, umfassend:

- einen Chip,
- auf dem Chip mindestens eine Vorrichtung nach einem der Ansprüche 1 bis 10 , und
- eine menschliche, pflanzliche oder tierische Zelle, die mit dem Gate in Kontakt steht.

## Claims

1. Apparatus for measuring biological and/or electronic properties of a sample, in particular for measuring cell parameters, in particular of individual human, plant or animal cells, comprising a field-effect transistor with a contacting device for contacting the sample with the gate of the field-effect transistor, a stimulation device for applying an AC voltage to the sample and a frequency-selective measuring instrument for measuring the amplitude and phase of a current flowing through the source-drain path of the field-effect transistor, wherein the field-effect transistor is an organic field-effect transistor (10) and the organic material of the field-effect transistor has a charge carrier mobility of 0.1 $cm^2/Vs$ to 3.6 $cm^2/Vs$.

2. Apparatus according to Claim 1, **characterized in that** the field-effect transistor has a gate width W/gate length L ratio ranging between 1 and 10 000, preferably ranging between 10 and 1000 and particularly preferably being 100 and/or the field-effect transistor comprises a finger structure or a spiral structure.

3. Apparatus according to either of the preceding claims, **characterized in that** the measuring area of the field-effect transistor is less than approximately 400 $\mu m^2$ and/or the diameter of the measuring area is less than approximately 28 $\mu m$ and/or the entire measuring area or only a part of same is optically transparent.

4. Apparatus according to any one of the preceding claims, **characterized in that** the gate length L is less than approximately 1 $\mu m$ and/or the field-effect transistor is structured by means of a nanostructuring method, such as electron beam lithography or deep UV lithography or nanoimprint lithography.

5. Apparatus according to any one of the preceding claims, **characterized in that** the source and drain feeds consist of an electrically conductive, preferably optically transparent material or a corresponding material mixture and are provided with, in particular electrically insulated by, a passivation layer made of, e.g., $SiO_2$, $Si_3N_4$ or parylene.

6. Apparatus according to any one of the preceding claims, **characterized in that** the organic semiconductor material (22) of the field-effect transistor is an optically transparent polymer such as, e.g., PEDOT:PSS, an oligomer or a 2D material such as, e.g., graphene or reduced graphene oxide, chalcogenide such as, e.g., $MoS_2$, or a combination or mixture of a plurality of same.

7. Apparatus according to any one of the preceding claims, **characterized in that** the organic semiconductor material (22) is coated with an insulation layer (24) such as, e.g., ethylene glycol for the purposes of electrically insulating the gate.

8. Apparatus according to any one of the preceding claims, **characterized in that** it comprises a substrate for the field-effect transistor made of preferably optically transparent material such as, e.g., glass or preferably optically transparent polymer material such as, e.g., Kapton, Neopulim® or the like, or a combination of same.

9. Apparatus according to any one of the preceding claims, **characterized in that** it is embodied as a sensor chip or as a constituent part of same.

10. Apparatus according to any one of the preceding claims, **characterized by** an excitation means for applying a further electric voltage or an electric current to the sample.

11. Array made of at least two apparatuses according to any one of the preceding claims.

12. Sensor chip comprising at least one apparatus according to any one of Claims 1 to 10.

13. Method for measuring biological and/or electronic properties of a sample, in particular for measuring cell parameters, in particular of individual human, plant and animal cells, comprising the steps of:

- arranging a sample in contact with the gate of an organic field-effect transistor of an apparatus according to any one of Claims 1 to 10,
- applying an AC voltage or an alternating current to the sample and
- measuring the amplitude and phase of a current flowing through the source-drain path of the field-effect transistor.

14. Method according to Claim 13, **characterized in**

**that** the AC voltage has a frequency between 0.1 Hz and 1 GHz, in particular between 1 Hz and 10 MHz, and/or the AC voltage has an amplitude of between 0.001 mV and 10 V, in particular between 1 mV and 100 mV.

15. Method according to either of Claims 13 and 14, **characterized in that** it is performed in a manner resolved in space and/or time.

16. Use of the apparatus according to any one of Claims 1 to 10, the array according to Claim 11 or the sensor chip according to Claim 12 for impedimetric analysis, in particular in a high throughput and/or individual cell method, of adherent cells in vitro.

17. Use of the apparatus according to any one of Claims 1 to 10, the array according to Claim 11 or the sensor chip according to Claim 12 for electronic analysis, in particular impedimetric analysis, in particular in a high throughput method, of stem cell differentiation.

18. Use of the apparatus according to any one of Claims 1 to 10, the array according to Claim 11 or the sensor chip according to Claim 12 for electronic biopsy, in particular in the high throughput method, of human, plant or animal tissue, in particular tumour tissue.

19. Use of the apparatus according to any one of Claims 1 to 10, the array according to Claim 11 and the sensor chip according to Claim 12 for analysing the effect of pharmaceutical substances and medicaments, in particular cancer medicaments such as, e.g., cytostatic agents and chemotherapeutics, or substances which influence the differentiation of stem cells.

20. Use of a method according to any one of Claims 13 to 15 for the electronic analysis, in particular impedimetric analysis, in particular in the high throughput method, of stem cell differentiation.

21. Use of a method according to any one of Claims 13 to 15 for the electronic biopsy, in particular in the high throughput method, of human, plant or animal tissue, in particular tumour tissue.

22. Use of a method according to any one of Claims 13 to 15 for analysing the cytotoxicity of substances and medicaments, in particular cancer medicaments such as, e.g., cytostatic agents and chemotherapeutics.

23. Use of a method according to any one of Claims 13 to 15 in combination with a transmitted light measuring method such as, e.g., a transmitted light microscopy method.

24. Cell-chip hybrid system for monitoring the activity of human, plant and animal cells and for analysing the cytotoxicity of substances and medicaments, in particular cancer medicaments such as, e.g., cytostatic agents, and for electronic biopsy, in particular in the high throughput method, of human and animal tissues, in particular tumour tissue, and for electronic analysis, in particular impedimetric analysis, in particular in the high throughput method, of stem cell differentiation, comprising:

- a chip,
- at least one apparatus according to any one of Claims 1 to 10 on the chip and
- a human, animal or plant cell which is in contact with the gate.

**Revendications**

1. Dispositif de mesure de propriétés biologiques et/ou électroniques d'un échantillon, en particulier pour la mesure de paramètres cellulaires, en particulier de cellules individuelles humaines, végétales ou animales, comprenant un transistor à effet de champ muni d'un dispositif de mise en contact pour mettre l'échantillon en contact avec la grille du transistor à effet de champ, un dispositif d'excitation pour soumettre l'échantillon à une tension électrique alternative, et un instrument de mesure sélectif en fréquence pour mesurer l'amplitude et la phase d'un courant passant sur le trajet source-drain du transistor à effet de champ,
le transistor à effet de champ étant un transistor à effet de champ organique (10) et la matière organique du transistor à effet de champ présente une mobilité des porteurs de charge comprise entre 0,1 $cm^2$/Vs et 3,6 $cm^2$/Vs .

2. Dispositif selon la revendication 1, **caractérisé en ce que** le transistor à effet de champ présente un rapport largeur de grille W / longueur de grille L compris dans la plage de 1 à 10000, de préférence dans la plage de 10 à 1000, et de plus grande préférence égal à 100, et/ou le transistor à effet de champ présente une structure de doigts ou une structure en spirale.

3. Dispositif selon l'une quelconque des revendications précédentes, **caractérisé en ce que** la surface de mesure du transistor à effet de champ est inférieure à environ 400 $\mu m^2$ et/ou le diamètre de la surface de mesure est inférieur à environ 28 $\mu m$, et/ou toute la surface de mesure ou seule une partie de celle-ci est optiquement transparente.

4. Dispositif selon l'une quelconque des revendications précédentes, **caractérisé en ce que** la longueur de grille L est inférieure à environ 1 $\mu m$, et/ou le tran-

sistor à effet de champ est structuré au moyen d'un procédé de nanostructuration, comme par exemple la lithographie à faisceau d'électrons ou la lithographie UV profonde ou la lithographie par nanoimpression.

5. Dispositif selon l'une quelconque des revendications précédentes, **caractérisé en ce que** les conducteurs d'alimentation de source et de drain sont composés d'un matériau électriquement conducteur, de préférence optiquement transparent, ou d'un mélange de matériaux correspondant, et sont munis d'une couche de passivation, comme par exemple en $SiO_2$, $Si_3N_4$ ou en parylène, en particulier isolés électriquement.

6. Dispositif selon l'une quelconque des revendications précédentes, **caractérisé en ce que** le matériau semi-conducteur organique (22) du transistor à effet de champ est un polymère optiquement transparent, comme par exemple le PEDOT:PSS, un oligomère ou un matériau 2D, comme par exemple le graphène ou de l'oxyde de graphène réduit, un chalcogénure, comme par exemple le $MOS_2$, ou une combinaison ou un mélange de plusieurs de ces éléments.

7. Dispositif selon l'une quelconque des revendications précédentes, **caractérisé en ce que** le matériau semi-conducteur organique (22) est recouvert d'une couche d'isolation (24), comme par exemple de glycol éthylénique, pour l'isolation électrique de la grille.

8. Dispositif selon l'une quelconque des revendications précédentes, **caractérisé en ce qu'**il présente un substrat pour le transistor à effet de champ, composé d'un matériau de préférence optiquement transparent, comme par exemple le verre, ou d'un matériau polymère de préférence optiquement transparent, comme par exemple le Kapton®, le Neopulim® ou similaires, ou d'une combinaison de ses éléments.

9. Dispositif selon l'une quelconque des revendications précédentes, **caractérisé en ce qu'**il est réalisé sous la forme d'une puce de capteur ou d'un composant de celle-ci.

10. Dispositif selon l'une quelconque des revendications précédentes, **caractérisé par** un moyen d'excitation pour soumettre l'échantillon à une tension électrique ou à un courant électrique supplémentaire.

11. Réseau d'au moins deux dispositifs selon l'une quelconque des revendications précédentes.

12. Puce de capteur, comprenant au moins un dispositif selon l'une quelconque des revendications 1 à 10.

13. Procédé de mesure de propriétés biologiques et/ou électroniques d'un échantillon, en particulier pour la mesure de paramètres cellulaires, en particulier de cellules individuelles humaines, végétales ou animales, comprenant les étapes consistant à :

- disposer un échantillon en contact avec la grille d'un transistor à effet de champ organique d'un dispositif selon l'une quelconque des revendications 1 à 10,
- soumettre l'échantillon à une tension alternative ou à un courant alternatif, et
- mesurer l'amplitude et la phase d'un courant passant par le trajet source-drain du transistor à effet de champ.

14. Procédé selon la revendication 13, **caractérisé en ce que** la tension alternative présente une fréquence comprise entre 0,1 Hz et 1 GHz, en particulier entre 1 Hz et 10 MHz, et/ou la tension alternative présente une amplitude comprise entre 0,001 mV et 10 V, en particulier entre 1 mV et 100 mV.

15. Procédé selon l'une quelconque des revendications 13 ou 14, **caractérisé en ce qu'**il est effectué en résolution spatiale et/ou en résolution temporelle.

16. Utilisation du dispositif selon l'une quelconque des revendications 1 à 10, du réseau selon la revendication 11 ou de la puce de capteur selon la revendication 12 pour une analyse impédimétrique, en particulier dans un procédé à haut débit et/ou un procédé à cellule unique de cellules adhérentes in vitro.

17. Utilisation du dispositif selon l'une quelconque des revendications 1 à 10, du réseau selon la revendication 11 ou de la puce de capteur selon la revendication 12 pour une analyse électronique, en particulier une analyse impédimétrique, en particulier dans un procédé haut débit d'une différenciation de cellules souches.

18. Utilisation du dispositif selon l'une quelconque des revendications 1 à 10, du réseau selon la revendication 11 ou de la puce de capteur selon la revendication 12 pour une biopsie électronique, en particulier dans un procédé haut débit, de tissus humains, végétaux ou animaux, en particulier de tissus tumoraux.

19. Utilisation du dispositif selon l'une quelconque des revendications 1 à 10, du réseau selon la revendication 11 et de la puce de capteur selon la revendication 12 pour une analyse du fonctionnement de substances pharmaceutiques et de médicaments, en particulier de médicaments anticancéreux, comme par exemple des agents cytostatiques et des agents chimiothérapeutiques, ou de substances qui affectent la différenciation des cellules souches.

20. Utilisation d'un procédé selon l'une quelconque des revendications 13 à 15 pour une analyse électronique, en particulier une analyse impédimétrique, en particulier dans un procédé haut débit d'une différenciation de cellules souches.

21. Utilisation d'un procédé selon l'une quelconque des revendications 13 à 15 pour une biopsie électronique, en particulier dans un procédé haut débit, de tissus humains, végétaux ou animaux, en particulier de tissus tumoraux.

22. Utilisation d'un procédé selon l'une quelconque des revendications 13 à 15 pour une analyse de la cytotoxicité de substances et de médicaments, en particulier de médicaments anticancéreux, comme par exemple des agents cytostatiques et des agents chimiothérapeutiques.

23. Utilisation d'un procédé selon l'une quelconque des revendications 13 à 15 en combinaison avec un procédé de mesure en transmission, comme par exemple un procédé de microscopie en transmission.

24. Système hybride cellule-puce pour surveiller l'activité de cellules humaines, végétales et animales et pour analyser la cytotoxicité de substances et de médicaments, en particulier de médicaments anticancéreux, comme par exemple des agents cytostatiques, et pour une biopsie électronique, en particulier dans un procédé à haut débit, de tissus humains, végétaux et animaux, en particulier de tissus tumoraux, et pour une analyse électronique, en particulier impédimétrique, en particulier dans un procédé à haut débit d'une différenciation de cellules souches, comprenant :

- une puce,
- sur la puce, au moins un dispositif selon l'une quelconque des revendications précédentes 1 à 10, et
- une cellule humaine, végétale ou animale en contact avec la grille.

Figur 1

Figur 2

Figur 3

Figur 4

Figur 5

Figur 6

Figur 7

Figur 8

Figur 9

Figur 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008061489 A1
- WO 200061489 A1
- WO 208061489 A1

**In der Beschreibung aufgeführte Nicht-Patentliteratur**

- Organic FET device as a novel sensor for cell bioelectrical and metabolic activity recordings. **SPANU A et al.** 2013 6TH INTERNATIONAL IEEE/EMBS CONFERENCE ON NEURAL ENGINEERING (NER). IEEE, 06. November 2013, 937-940
- **M. YAMASHITA ; C. OTANI ; M. SHIMIZU ; H. OKU-ZAKI.** Effect of solvent on carrier transport in poly(3,4-ethylenedioxythiophene)/poly(4-styrenesulfonate) studied by terahertz and infraredultraviolet spectroscopy. *Appl. Phys. Lett.,* 2011, vol. 99, 143307
- **DONALD A. NEAMEN.** Semiconductor Physics and Devices: Basic Principles. McGraw-Hill Publ, 2002
- **DONALD A. NEAMEN.** Semiconductor Physics and Devices: Basic Principles. McGraw-Hill Publ, 2002
- **M. PABST ; G. WROBEL ; S. INGEBRANDT ; F. SOMMERHAGE ; A. OFFENHÄUSSER.** Solution of the Poisson-Nernst-Planck equations in the cell-substrate interface. *European Physical Journal E - Soft Matter,* 2007, vol. 24, 1-8